# EUROPEAN PATENT SPECIFICATION

(11) **EP 3 517 606 B1**
(45) Date of publication and mention of the grant of the patent: **28.01.2026**
(21) Application number: 17853510.0
(22) Date of filing: 25.09.2017
(51) Int. Cl.: C12N 5/071, C12N 5/073, A61K 35/33, A61K 35/36, A61P 17/02

(54) **METHOD FOR PREPARING A SUPPLEMENT FROM MESENCHYMAL CELL CULTURES OF WHARTON'S JELLY AND USES OF SAME**
VERFAHREN ZUR HERSTELLUNG EINES SUPPLEMENTS AUS MESENCHYMALEN ZELLKULTUREN VON WHARTON-SULZE UND VERWENDUNGEN DAVON
PROCÉDÉ DE PRÉPARATION D'UN TISSU À PARTIR DE CULTURES DE CELLULES MÉSENCHYMATEUSES DE GÉLATINE DE WHARTON ET UTILISATIONS DE CE DERNIER

(30) Priority: 23.09.2016 MX 2016012375
(43) Date of publication of application: 31.07.2019
(73) Proprietor: Universidad Nacional Autónoma De México, 04510 Ciudad de México (MX)
(72) Inventor: CASTELL RODRÍGUEZ, Andrés Eliú, C.P. 04630 Ciudad de México (MX); HERRERA ENRÍQUEZ, Miguel Ángel, C.P. 16200 Ciudad de México (MX); PIÑÓN ZÁRATE, Gabriela, C.P. 03300 Ciudad de México (MX); JARQUÍN YÁÑEZ, Katia, C.P. 37850 Guanajuato (MX); CHAIRES ROSAS, Casandra, C.P. 02650 Ciudad de México (MX); ARELLANO OLIVARES, Rosa María, C.P. 01260 Ciudad de México (MX)
(74) Representative: Clarke, Modet y Cía., S.L.
(86) International application number: PCT/MX2017/000103
(87) International publication number: WO 2018/056798

(56) References cited:
- US-A1- 2002 172 705
- US-A1- 2012 195 969
- US-A1- 2013 302 285
- VELANDER P ET AL: "Cell Suspensions of Autologous Keratinocytes or Autologous Fibroblasts Accelerate the Healing of Full Thickness Skin Wounds in a Diabetic Porcine Wound Healing Model", JOURNAL OF SURGICAL RESEARCH, vol. 157, no. 1, November 2009 (2009-11-01), pages 14 - 20, XP026694373, ISSN: 0022-4804, DOI: 10.1016/J.JSS.2008.10.001
- FONG CHUI-YEE ET AL.: "Human Wharton's Jelly Stem Cells and Its Conditioned Medium Enhance Healing of Excisional and Diabetic Wounds", JOURNAL OF CELLULAR BIOCHEMISTRY, vol. 115, 2014, pages 290 - 302, XP055367464, ISSN: 0730-2312
- MIRANDA JOANA P ET AL.: "The Human Umbilical Cord Tissue-Derived MSC Population UCX (R) Promotes Early Motogenic Effects on Keratinocytes and Fibroblasts and G-CSF-Mediated Mobilization of BM- MSCs When Transplanted In Vivo", CELL TRANSPLANTATION, vol. 24, no. 5, 30 November 2014 (2014-11-30), pages 865 - 877, XP055498191, ISSN: 0963-6897
- IMING YANG ET AL.: "Umbilical cord-derived mesenchymal stem cells: strategies, challenges, and potential for cutaneous regeneration", FRONTIERS OF MEDICINE, vol. 6, no. 1, 31 March 2012 (2012-03-31), Heidelberg, pages 41 - 47, XP035038008, ISSN: 2095-0225
- SCHNEIDER REBEKKA K ET AL.: "Long-term survival and characterisation of human umbilical cord-derived mesenchymal stem cells on dermal equivalents", DIFFERENTIATION, vol. 79, no. 3, 28 February 2010 (2010-02-28), pages 182 - 193, XP026954423, ISSN: 0301-4681
- EDWARDS SANDRA S ET AL.: "Functional analysis reveals angiogenic potential of human mesenchymal stem cells from Wharton's jelly in dermal regenera", ANGIOGENESIS, vol. 17, no. 4, 13 April 2014 (2014-04-13), pages 851 - 866, XP035398249, ISSN: 0969-6970

## Description

### FIELD OF THE INVENTION

The present invention pertains to the field of tissue engineering. In particular, the present invention concern to a method for preparing a supplement from mesenchymal cell cultures of Wharton's jelly and uses of same.

### BACKGROUND OF THE INVENTION

The cutaneous trauma cause diverse conditions that put at risk the health of patient, between these conditions are included dehydration, loss of electrolytes and proteins in the wound site, at the same time that the wound remains exposed to bacterial infections. In the last decades diverse methods has been developed, allowing replacement of the main components of skin, epidermis, and dermis, with biological equivalents produced *in vitro.*

Between the methods employed for preparing epidermal equivalents, the most common corresponds to describe in the 70's decade by Rheinwald James G and Green (Serial cultivation of strains of human epidermal keratinocytes: the formation of keratinizing colonies from single cells, 1975). The method of Rheinwald and Green have a high reliability, studies have been performed (Benathan and Labidi-Ubaldi, Substitus épidermiques et dermo-6pidermiques vivants pour le traitement des grands brûlés, 1998) where from 65 performed preparations, only anomalies were detected in four cases in the dermic equivalents prepared, two of those cases corresponded to cultures in which was poor stratification, in the other two the cells revealed signs of apoptosis.

In the clinic practice, the method of Rheinwald and Green presents difficulties such as the high fragility from epidermal grafts cultivated in absence from dermal equivalents and high cost from transplants (Miguel Concha, Production from autologous dermo-epidermal equivalents for treatment of great burns and keloid scars, 2002). On the other hand, the inclusion of elements from animal origin in the process of elaboration from Rheinwald and Green for epidermal equivalents can attend with the persistence of mouse fibroblasts, as well as residuals of bovine fetal serum in the finalized epidermal equivalent (Cairns BA, Xenogenic mouse fibroblast persists in human cultured epidermal grafts: a possible mechanism of graft lost, 1995), which can cause the rejection of it because the immune response from patient.

To build epidermal equivalents both cutaneous and/or dermal, cells 3T3 have been used to induce the proliferation of keratinocytes in culture. The cells 3T3 are mouse embrionary fibroblasts, serving as feeder cells (humans or animals) or "nurses" (Rheinwald JG, Green H., Serial cultivation of strains of human epidermal keratinocytes: the formation of keratinizing colonies from single cells, 1975). This method has been widely used to grow keratinocytes.

Briefly, the previous method comprises to grow the obtained cells from a biopsy in presence of irradiated cells 3T3, subsequently; the culture of keratinocytes washes and collects. The previous methodology has allowed to obtain large quantities of keratinocytes, in order to construct epidermal equivalents including cutaneous equivalents or dermal equivalents used to treat patients with second or third degree burns (Llames S, Garcia E, Garcia V, del Rio M, Larcher F, Jorcano JL, et al. Clinical results of an autologous engineered skin, 2006).

On the other hand, there is evidence that the use of mesenchymal cells applied directly in wounds promotes the recovery by an indirect mechanism, delivering diverse molecules that may have a therapeutic effect, for example, growth factors and other cytokines, while the mesenchymal cells can differentiate to fibroblasts (Chui-Yee Fong, Human Wharton's Jelly Stem Cells and Its Conditioned Medium Enhace Healing of Excisional and Diabetic Wounds, 2014). The mesenchymal cells obtained from Wharton's jelly secrete different factors, which can induce the proliferation of keratinocytes and fibroblasts in culture. The use of conditioned media to grow several cell types has gained ground strongly, in which it has been observed that resident cells of a tissue can modulate the secretory activity from mesenchymal cells and these in turn induce the differentiation to cells from tissue in question (Vidya Gopakumari, Nivedita Chatterjee, Sowmya Parameswarani, Subramanian Nirmala and Subramanian Krishnakumar, 2016). However, a history in the production of use epidermal equivalents from does not exists, whether cutaneous and/or dermal so that promotes the delivery of growth factors necessary to stimulate the cellular proliferation *in vitro* from several cells of cutaneous system, to perform epidermal equivalents whether cutaneous and/or dermal.

In the state of the art already disclosed alternative methods for preparing epidermal equivalents to avoid the use of animal elements, for example using autologous or allogenic human fibroblasts to promote the growth and differentiation of keratinocytes during the elaboration from cutaneous equivalent (Mazlyzam, Reconstruction of living bilayer human skin equivalent utilizing human fibrin as a scaffold, 2007). Even though these methods avoid the risk of allergic reactions to animal components (zoonosis), do not reduce significantly the cost to patient. Then, there is a need to have alternative methods for preparing epidermal equivalents offering different advantages.

### BRIEF DESCRIPTION OF THE INVENTION

Here is described a method for preparing a supplement that when used in the elaboration of epidermal equivalents, permits the removal from risk of zoonosis and offers a decrease in the manufacturing costs of same.

Additionally, the supplement obtained by the method of present invention can be used as additive in the cell cultures from cutaneous system, to elaborate a cell suspension of fibroblasts and to use in the treatment of diseases, defects, cutaneous disorders as result from cutaneous aging.

The application of cultivated fibroblasts using the supplement obtained by the method developed increases the cellular activation, promoting an increase from mechanical stress in the extracellular matrix, inducing an elongation from fibroblasts and the stimulation in the collagen synthesis, release of growth factors, inhibition from metalloproteinase secretion, and increasing the secretion from inhibitor factors of the same.

The supplement is prepared from the supernatant of mesenchymal cell cultures obtained from Wharton's jelly cultivated in specific conditions. The supplement includes growth factors necessary to favor and increases the cellular proliferation *in vitro* for several cells from cutaneous system.

### BRIEF DESCRIPTION OF DRAWINGS

The invention is illustrated below, without limitation, with reference to described examples and figures:

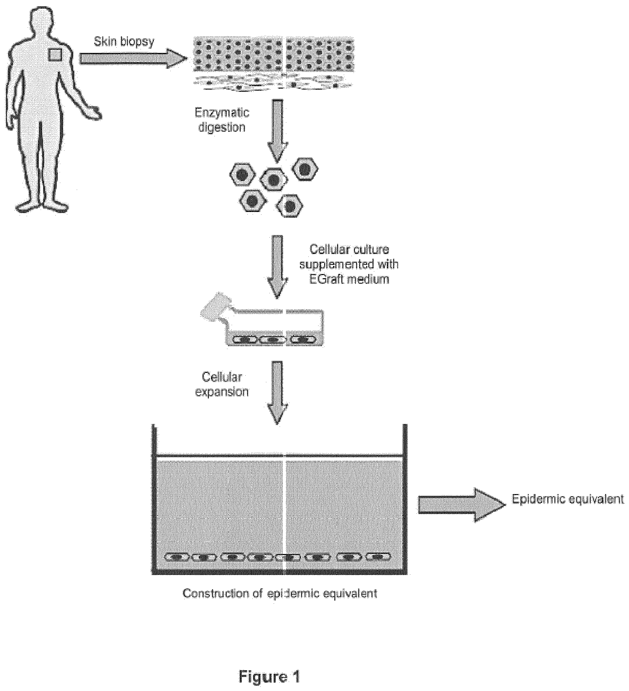

The Figure 1 shows the flowchart to elaborate an epidermal equivalent from autologous cells of patient.

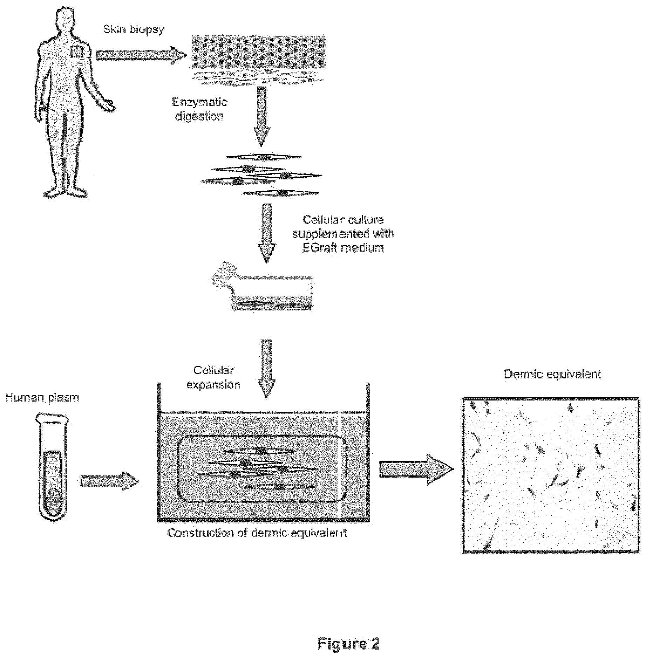

The Figure 2 shows the flowchart to elaborate a dermal equivalent from autologous cells of patient.

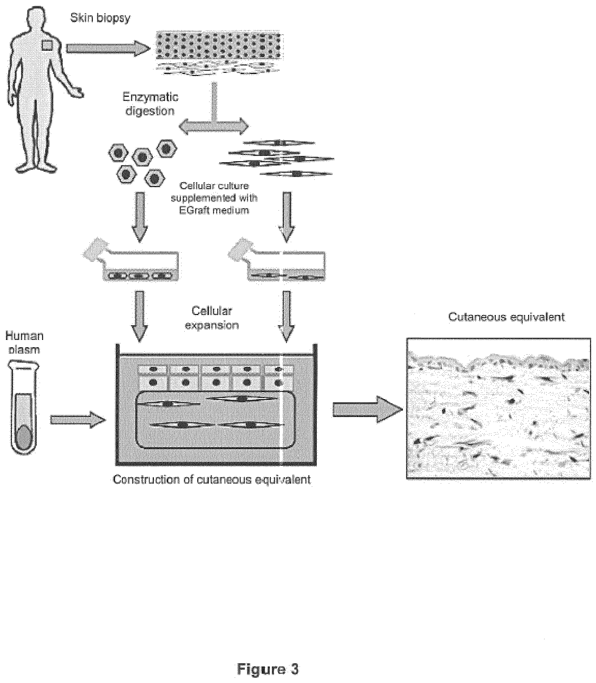

The Figure 3 shows the flowchart to elaborate a cutaneous equivalent from autologous cells of patient.
The Figure 4 shows the graphic which compares the reduction from area of ulcers in patients treated with a dermal equivalent elaborated by the method of the invention (light gray line) and a conventional hydrocolloid dermal dressing (dark gray line).
The Figure 5 shows a simultaneous comparative graph in: i) the area in cm² of wound reduction, ii) the area in percentage of wound reduction, and iii) the closing velocity (cm²/week) for ulcers in patients treated with a dermal equivalent generated using the supplement elaborated by the developed method (columns of light gray color) and a conventional hydrocolloid dermal dressing (columns of dark gray color).
The Figure 6A shows an ulcer wound of 17.7 X 4 centimeters in the patient. The Figure 6B shows the closed wound in the patient after seven weeks of treatment employing the dermal equivalent generated using the supplement elaborated by the developed method.

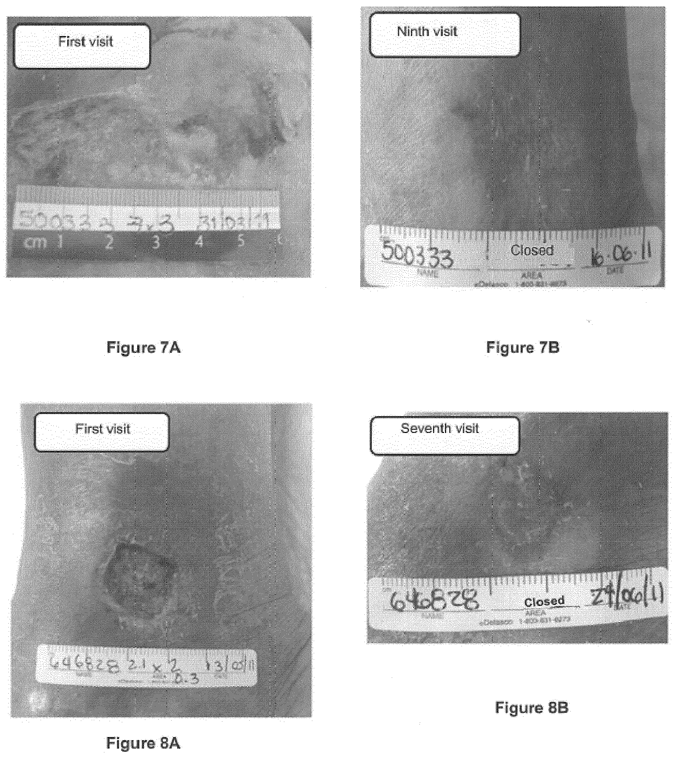

The Figure 7A shows an ulcer wound de 7 X 3 centimeters in the patient. In the Figure 7B shows the closed wound in the patient after nine weeks of treatment employing the dermal equivalent generated using the supplement elaborated by the developed method.
The Figure 8A shows an ulcer wound of 2.1 X 2 centimeters in the patient. The Figure 8B shows the closed wound in the patient after seven weeks of treatment employing the dermal equivalent generated using the supplement elaborated by the developed method.

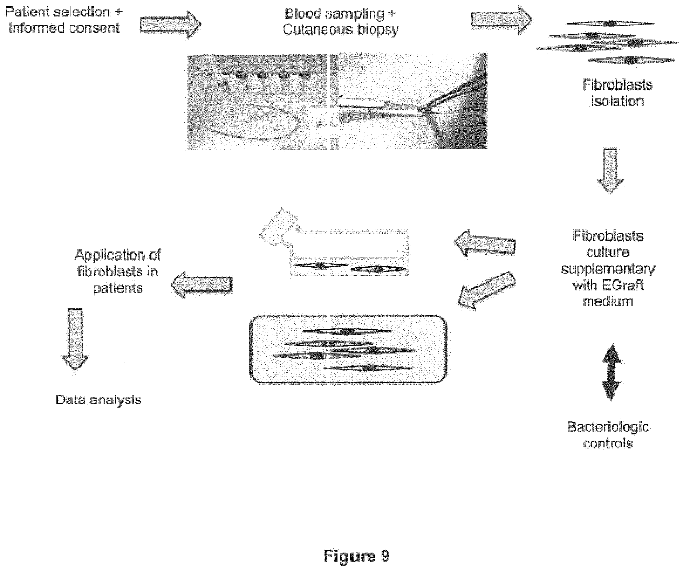

The Figure 9 shows a simplified flowchart to elaborate a cellular suspension of fibroblasts to be applied in diseases, defects and cutaneous disorders as result from skin aging from autologous cells of patient.

### DETAILED DESCRIPTION OF THE INVENTION

The present invention is related with the production of a supplement from mesenchymal cell cultures of Wharton's jelly and its use in the development of cell cultures from cutaneous system *in vitro.*

As used in the present invention, the cells of cutaneous system comprise fibroblasts, keratinocytes, dendritic cells, monocytes or combinations of the same, which can be cultivated in an isolated manner. The supplement obtained by the method in the present invention can be used in the expansion of cutaneous cell populations or incorporated in culture means for the same, independently from intended use of the cells.

In a first aspect, the present invention provides a method to obtain a supplement from mesenchymal cells of Wharton's jelly.

In a modality, the method to obtain the supplement comprises:
a) Cultivate mesenchymal cells of Wharton's jelly in a suitable culture medium with development factors, namely epidermal growth factor (EGF) and basic fibroblast growth factor (bFGF), to reach a suitable confluence;
b) Harvest the cultivated cells from step a) and cultivate in DMEM 50% - F12 50% with fetal bovine serum, without development factors;
c) Collect the conditioned mean from step b);
d) Repeat the steps a) and b), at least once; and
e) Concentrate the conditioned mean and sterilize by filtration.

The mesenchymal cells of Wharton's jelly used in the present invention are obtained from collect a fragment of umbilical cord, under aseptic conditions, subsequently the size of umbilical cord is reduced by a medical and enzymatic treatment.

As used in the present invention, "Cultivate mesenchymal cells of Wharton's jelly" refers to cultivate treated fragments from umbilical cord in a suitable culture mean to reach a suitable confluence.

As used in the present invention "Harvest the cultivated cells" refers to separate the previous cultivated cells from culture mean by an enzymatic treatment; wash the previous cultivated cells; and seeds the previous cultivated cells in a new suitable culture mean during a determined time, preferably 48 hours. As used in the present invention, "harvest the cultivated cells" is equivalent to "step".

The cell culture in accordance with the steps a) and b), is repeated preferably at least once, at least 2 times, at least 3 times, at least 4 times, at least 5 times, at least 6 times, at least 7 times, at least 8 times, at least 9 times.

As used in the present invention, "the conditioned mean" refers to culture mean obtained from seed or steps, that is found free of cells. Besides contain the proper components of their formulation, contains the secretion products (proteins, hormones, development factors, cytokines, and signaling molecules) that are produced and delivered by the cells in question during its culture.

As used in the present invention, the term "supplement" corresponds to the conditioned mean by the mesenchymal cells concentrated and sterilized. The supplement can be used in a culture mean.

In a preferred modality, the supplement from present invention comprises several cytokines, for example, the compounds Actine, Annexin A1, Amyloid beta A4, ATP synthase sub-unity-B, Cadherin 2, CD44, Collagen chain alpha-1(VIII), EGF, derived factor of epithelium pigmentary, Fibronectin, FGF-2, Inhibin chain Beta, Integrin B1, Interleukin 6, ILGF-bp4, ILGF-bp7, Keratin 2, Laminar sub-unity Alpha-2, Lipoprotein lipase, MAPK1, Myosin 9, Moesin, M-CSF, MPI-1, PI-2, Nexin derived of glia, NDP-kA, NDP-kB, PAI-1, PDGF sub-unity A, Peroxyredoxin 1, Peroxyredoxin 2, Complementary protein 5 of reparation to XR , protein ih-h3 induced by TGFB, Purine nucleoside phosphorilase, Sulfhydryl oxidase, SPARE, Substrate 1 from botulinum toxin related to Ras, Substrate 2 from botulinum toxin related to Ras, Thrombospondin, Tropomyosin alpha 1, TGFB, TGFB-R1, TGFB-R2, VEGF A, and Vinculin.

The term "mesenchymal cells of Wharton's jelly" refers to multi-potential cells from mesoderm obtained from umbilical cord.

The term "confluence" refers to the number of cells in a cell culture and is referred to grade in which the cells cover the surface of a solid culture mean or volume of a cellular culture. The confluence is measured in % with respect to occupied total volume.

As described in the present invention, the term "suitable confluence" refers to a suitable percentage (%) to be used in a process, for example, in the elaboration from supplement, elaboration from dermal equivalent or cutaneous equivalent in accordance with the present invention. Preferably, such percentage is greater than 60%, more preferably, is greater than 80%.

The term "suitable culture mean" refers to a culture mean for mesenchymal cells, for example, a DMEM-F12 mean (DMEM 50% - F12 50%) supplemented with 10% bovine fetal serum 10% and antibiotics. The suitable culture mean is supplemented with development factors, namely epidermal growth factor (EGF) and basic fibroblast growth factor (bFGF).

In other aspect, the present invention provides methods to obtain an epidermal equivalent using the supplement from present invention. As used in the present invention, the epidermal equivalents refer to epithelial cells laminates grown over a culture. The epidermal equivalents are useful to be applied in patients with second degree superficial burns or with cutaneous chronic ulcers of different etiology. The epidermal equivalents can be dermal or cutaneous equivalents.

As used in the present invention, the term "dermal equivalent" refers to a tissue construed by tissue engineering comprising a cellular layer of fibroblasts and an extracellular matrix organized similarly to dermal tissue in the skin. The fibroblasts are located placed within a scaffold of natural, synthetic or composite biomaterials.

As used in the present invention, the term "cutaneous equivalent" refers to a tissue constructed by tissue engineering comprising an internal layer and an external layer, in which the internal layer comprises the dermal complex, and the external layer comprises the epidermal complex constituted by a monolayer of keratinocytes. Both layers are organized similarly to dermis and epidermis in the skin, respectively. In a modality, the external layer consists of stratified plane epithelium with stratum corneum. The epidermal equivalent is located placed over the dermal equivalent constructed with natural, synthetic or composite biomaterials.

In a preferred modality, the method to obtain the dermal equivalent comprises:
a) Cultivate autologous or allogenic fibroblasts in a culture mean containing the supplement from present invention to obtain a suitable confluence; and
b) Produce the dermic equivalent.

The fibroblasts used in the method to obtain the dermal equivalent can be obtained from autologous or allogenic cells from cutaneous system, from which the fibroblasts of dermis are separated by an enzymatic treatment.

As used in the present invention "cultivate fibroblasts" refers to maintain fibroblasts in a culture mean comprising the supplement from present invention to reach a suitable confluence; separate previous cultivated cells from culture mean by an enzymatic treatment; wash the previous cultivated cells; and reseeds the cells in a culture mean comprising the supplement from present invention to reach a suitable confluence for the construction from dermic equivalent. The supplement from present invention is used in a concentration from of 5% to 50% with respect to total volume form culture mean.

As used in the present invention, "Produce the dermic equivalent" refers to place the fibroblasts cultivated previously in a solution containing fibrinogen and prothrombin in presence of antifibrinolytics, sodium chloride and calcium chloride, to form a cellular suspension; place the cellular suspension in a container and incubate to its gelation; and place the gel over a scaffold of natural, synthetic or composite biomaterials with adhesive biocompatible.

In a preferred modality, the method to obtain the cutaneous equivalent comprises:
a) Cultivate autologous or allogenic keratinocytes in a culture mean containing the supplement from present invention to obtain a suitable confluence; and
b) Produce the cutaneous equivalent.

As used in the present invention "cultivate keratinocytes" refers to cultivate keratinocytes in a culture mean comprising the supplement from present invention to reach a suitable confluence; separate previous cultivated cells from culture mean by an enzymatic treatment; wash the previous cultivated cells; and reseeds the cells in a culture mean comprising the supplement from present invention to reach a suitable confluence, necessary to the construction the cutaneous equivalent. The supplement from present invention is used in a concentration of 5% al 50% with respect to total volume from culture mean.

As used in the present invention "Produce the cutaneous equivalent" refers to seed and cultivate the keratinocytes previously cultivated over gelled fibroblasts in a culture mean added with the supplement from present invention to reach the suitable confluence; and place over a scaffold of natural, synthetic or composite biomaterials with adhesive biocompatible. In a preferred modality, the fibroblasts used to elaborate the cutaneous equivalent, are obtained according to the elaboration method of dermic equivalent. The supplement from present invention is used in a concentration of 5% to 50% with respect to total volume from culture mean.

The term "autologous system" comprises an epithelial and dermal cells system obtained from the same person to which the tissue will be grafted. The term "allogenic system" comprises epithelial and dermic cells obtained from a different person to which the tissue will be grafted.

Dermal equivalents obtained by the method of the present invention may be used to treat diseases, defects, dermal disorders, or wounds. The dermal equivalents from present invention are useful to induce in the patient a regeneration from conjunctive tissue, to subsequently promote the reepithelialization. The use of dermal equivalents from present invention consists in apply directly the dermal equivalent over the wound bed at least once a week to regenerate the dermal and epidermal tissues from patient, what is achieved un a period from 2 to 14 weeks treatment, preferably 4 to 8 weeks.

The candidates to receive the treatment with dermal equivalents comprise patients presenting diseases, defects, dermal disorders, or wounds, for example, ulcers, chronic wounds, vascular, arterial, venous, and lymphatic, of decubitus or diabetic foot. The patient must be free of infection in wound and in case of being diabetic with the controlled glycaemia. To apply the dermic equivalent, the wound must be washing by the use of antiseptic solution to subsequently debridement from lesion. After preparing the area of lesion, the dermal equivalent is directly implanted over the injured tissue, subsequently is covered with an insulating bandages to prevent its detachment, keeping in that way by a sufficient time to reach the closure of the wound.

Cutaneous equivalents obtained by the method of the present invention may also be used in the treatment from diseases, dermal defects, cutaneous disorders or wounds when the thickness from condition is partial or with an extension requiring graft. The use from cutaneous equivalent is applicable for example, when the dermal defect is a nevus, tattoo, hypertrophic scar, keloid or burn scar or of a size large enough to be surgically removed. The use from cutaneous equivalent can be applied for the case of second or third degree burns from a size large enough to be surgically removed.

The implantation from cutaneous equivalent is performed by a surgical procedure, in which the cutaneous equivalent is placed as a graft immediately after of retirement from affected tissue, fixing by using sutures, surgical staples or a suitable adhesive, to subsequently cover with un an insulating bandage to avoid its detachment. Once implanted the cutaneous equivalent, the affected zone remains covered by a sufficient time to reach the closure of the wound.

In other modality, a method to manufacture a cellular suspension of fibroblasts is provided. The method comprises:
a) Cultivate fibroblasts, in a culture mean comprising the supplement from present invention to reach a suitable confluence;
b) Incorporate the fibroblasts from step a) in a solution of autologous blood serum.

In other modality, the obtained supplement from developed method or cellular suspension of fibroblasts described in the above paragraph, can be used to favor the growth and activation *in vitro* from cells of cutaneous system which can be inactive by age, for example in the skin of adult patients with fine, moderate to deep, or deep wrinkles; loss of dermal tone and subcutaneous tissue; injuries in the skin such as atrophic scares as acne marks grade I (macular), II (mild), III (moderate), and IV (serious) and injuries of cutaneous caused by red or white stretch marks; pigmentary injuries from type solar or senil lentigo and simple lentigo, melasma and melanodermias from different etiology; injuries of acne; photo-aging severe classified with Fiztpatrick's scale from I, II, or III, in Glogau's scale fom 1, 2, 3, or 4 and in SCINEXA's scale from 0, 1, 2, or 3 characterized in that loss of elasticity and tissue tone, this because fibroblasts gradually losing the capacity to synthetize some elements from dermic extracellular matrix, so that can be obtain autologous fibroblasts from such patients and cultivate in presence from supplement elaborated according with the developed method in a concentration from between 5% to 50%, 5% to 50% with respect to total volume from culture mean, promoting a reactivation from cells restoring its synthesis and secretion capacities of dermic matrix elements so they can re-implanted by intradermal injections in areas with major damage by photo-aging.

### EXAMPLE 1 - Production of conditioned mean from culture of mesenchymal cells of Warthon's jelly from umbilical cord.

The conditioned mean was obtained from following procedure:
a) Collect, under aseptic conditions, a fragment of between 10 to 15 cm of umbilical cord;
b) Immerse the umbilical cord in a tube Corning of 50 ml with DMEM medium, supplemented with a solution 100X of antibiotics (penicillin [10,000 U/mL] and streptomycin [10,000 µg/mL]) and antifungal (fungizone [25 µg/mL]);
c) Cut up the umbilical cord in fragments of 1 cm length;
d) Incubate the fragments of umbilical cord in culture mean supplemented with the enzymes: collagenase and hyaluronidase during 30 min, promoting a partial digestion from Warthon's jelly;
e) Place the fragments of umbilical cord in culture plates with el DMEM-F12 medium (DMEM 50% - F12 50%) supplemented with 10% bovine fetal serum and antibiotics;
f) Perform the change of culture mean by a fresh mean, such as described in the step e each 48 hours. Repeat to reach confluence greater than 90%;
g) Harvest the cells by its incubation with a mean supplemented with Trypsin/EDTA;
h) Inactivate and remove the trypsin by washing and centrifugation;
i) Harvest the obtained cells of the harvest in a proportion from up 1 in 10 in new culture containers such as expands the cellular colony;
j) Cultivate the cells in culture containers in a culture mean of DMEM supplemented with factor for epidermal growth (EGF), basic factor of fibroblasts growth (FGFb), bovine fetal serum and antibiotics and cultivate to reach a confluence from 90%;
k) Wash the cultivated cells from step j) tree times with culture medium DMEM not supplemented to remove the growth factors exogenously added and maintain in culture with DMEM not supplemented during 48 hours;
l) Once elapsed 48 hrs, collect the conditioned mean by the cells from step k);
m) After collect the conditioned mean, repeat the harvest processes, inactivation of trypsin, re-seeds, wash, culture and collect of supplement from steps g), h), i), j), k) and I) to step number 6;
n) By centrifugation sediment the cellular debris suspended in each conditioned means collected in the step m);
o) Concentrate the conditioned mean in a concentration of 10X by centrifugation in tubes Amicon;
p) Sterilize by filtration the concentrated conditioned mean with PVDF filter of low affinity to proteins with pore size of 0.22 um;
q) Preserve the conditioned mean to its storage by ultra-freeze to -80°C; and
r) Use the conditioned mean in cultures of keratinocytes, fibroblasts and other cells from cutaneous system to induce a proliferation of the same to a concentration of between 5% and 50% diluted in culture means.

### EXAMPLE 2 - Production of dermal equivalent.

A dermal equivalent was produced by the following protocol:
a) Cultivate fibroblasts in suitable culture conditions added with the supplement in a concentration of between 5% and 50% diluted in culture means;
b) Perform the change from culture mean by fresh culture mean, each 48 hours. Repeat to reach confluence greater than 80%;
c) Harvest the cells by its incubation with a supplemented means with Trypsin/EDTA;
d) Inactivate and remove the trypsin by washing and centrifugation;
e) Harvest the cells obtained from harvest in a proportion from up 1 in 10 in nevus culture containers such that expands the cellular colony;
f) Incorporate the fibroblasts from step e) in an aqueous solution containing fibrinogen and prothrombin obtained from human blood plasma in presence of antifibrinolytics, sodium chloride and calcium chloride to form a cellular suspension;
g) Place the cellular suspension from step f) in a culture container and incubate up the plasm to gels;
h) Cultivate the gelled dermal equivalent in culture with conditioned means during 5 days changing the fresh conditioned cultured means fresco each 48 hrs to a concentration of between 5% and 50% diluted in culture means; and
i) Place over the dermal equivalent a sterile nylon mesh and adhere over the dermal equivalent with a biocompatible adhesive, as such surgical methacrylate.

### EXAMPLE 3 - Production of cutaneous equivalent.

A cutaneous equivalent was produced by the following procedure.
a) Collect 1 cm² of skin of patient and transfer to a sterile tube with antiseptic solution of benzalkonium chloride or 70% ethanol and incubate during 30 seconds;
b) Apply tree consecutive washes with culture means supplemented with antibiotics;
c) Cut up the skin specimens in fragments of approximately 2 mm and transfer to a tube with supplemented means with dispase and incubate during 18 hrs to 4°C;
d) Mechanically separate the dermis from epidermis;
e) Transfer the epidermal laminates to a tube with trypsin-EDTA and incubate during 30 minutes at 37°C in continue agitation;
f) Collect the supernatant and place apart the dermic laminate;
g) From supernatant, obtain by centrifugation a epidermal celular suspension;
h) Use the cells obtained for the culture of keratinocytes;
i) Transfer the dermal laminate from step g) to a new tube with means supplemented with the collagenase enzyme and cultivate during 4 hrs at 37 °C in continue agitation to complete digestion of dermal tissue;
j) Obtain the supernatant and centrifuge to obtain a dermal celular suspension including fibroblasts;
k) Place the fibroblasts in activation and proliferation conditions in a culture means added with the supplement from present invention to a concentration of between 5% and 50% diluted in culture means and cultivate changing with the same means each 48 hrs to reach a confluence greater than 80%;
l) Harvest the cells by incubation with a supplemented means with Trypsin/EDTA;
m) Wash and centrifuge to inactivate and remove the trypsin;
n) Seed the cells obtained from harvest in a rate from up 1 in 10 in new culture containers such as to expands the cellular colony;
o) Add the keratinocytes from step d) to dermal complex from step c) to form the cutaneous equivalent;
p) Repeat the re-seed to obtain the desired cellular density to cutaneous equivalent without expand the cells beyond from step 8; and
q) Place over the cutaneous equivalent a sterile nylon mesh and adhere over the cutaneous equivalent with a biocompatible adhesive, such as surgical methacrylate.

### EXAMPLE 4 - Use from dermal equivalent in patients with ulcers.

The dermal equivalent obtained was used in the treatment of ulcers, in accordance with the following procedure.
a) Select a patient presenting ulcers of long progression, either of vascular origin, by pressure, or diabetic foot;
b) Verify that the patient be free of infection in the ulcer and in case of being diabetic, with the glycaemia controlled;
c) Wash the ulcer by the use of antiseptic solutions;
d) Debride the ulcer removing any debris of necrotic tissue;
e) Implant the dermal equivalent from area of injury directly over the ulcerated tissue;
f) Cover with an insulating bandage;
g) Maintain the bandage during seven days; and
h) Wash the wound with antiseptic solutions at least once a week, to reach the closure of the wound.

### EXAMPLE 5 - Use from cutaneous equivalent in patients with burns from autologous cells.

The cutaneous equivalent obtained according with the present invention was used in the treatment from patients with burns, according with the following procedure.
a) Select a patient presenting second or third degree burns in an area of the skin that in according with the clinical judgment that considers the specialist physician, have indication to apply a graft;
b) Collect an specimen of complete skin (dermis and epidermis) from patient;
c) Isolate the epidermal from dermal layer by enzymatic digestion;
d) Obtain independently keratinocytes and fibroblasts;
e) Cultivate and expand the keratinocytes and fibroblasts in presence of the conditioned mean, to obtain the cellular density necessary to construct the superficial extension required to the graft;
f) Prepare the patient suffering of second or third degree burns to a surgical procedure in which the cutaneous equivalent will implant as a graft;
g) Remove the burned tissue by an elective surgical procedure;
h) Place the cutaneous equivalent as a graft immediately after removal from burned tissue;
i) Fix the cutaneous equivalent by the use of sutures, surgical staples or a suitable adhesive;
j) Cover the cutaneous equivalent with an insulating bandage to avoid its detachment;
k) Maintain covered the zone in which the cutaneous equivalent was placed and review between the days 5 and 7 later to verify its implantation; and
l) Handle with care the implant from cutaneous equivalent and if it is necessary clean the area.

### EXAMPLE 6 - Comparative experiment of application from dermal equivalent generated using the supplement elaborated by the developed method and a conventional hydrocolloid dermal equivalent.

Were recruited 36 patients from which is consent was obtained to participate in the experiment, each patient was randomly assigned to treatment with the dermal equivalent generated using the supplement elaborated by the developed method or a conventional hydrocolloid dermal equivalent so that 18 patients were assigned to each treatments.

In the Table 1 the general data from patients participating in the experiment are shown including gender, age, evolution, consumption of NSAIDs, comorbidities and size from wound to each of the two compared groups. A statistical comparison from mean values is included to each considered parameter in accordance with the statistic test applied, a P value greater than 0.05 indicate the existence of statistically significant differences between both groups. As shown in the P column from Table 1, statistically significant differences do not exist between both groups for the measured parameters.

In the Table 2 the characteristics of ulcers are shown for each patients group including islands of epithelium, granulation, fibrin, exudate and pain of each from two groups compared. Same in the Table 1, a statistic comparison is included from the mean values for each considered parameter. As shown in the P column from Table 2, statistically significant differences do not exist between the characteristics from ulcers between both groups.

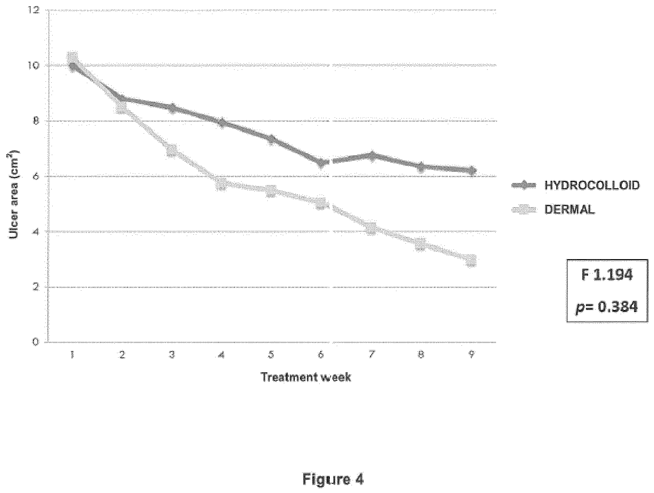

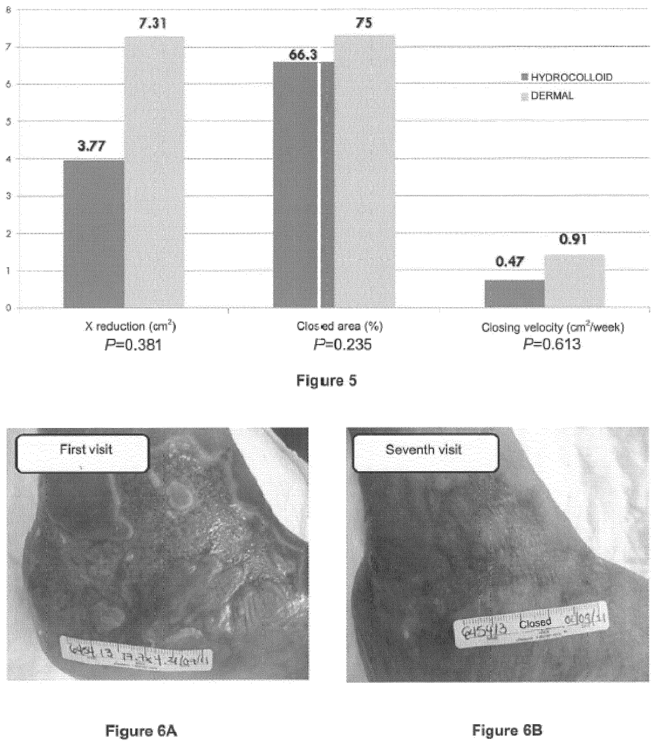

In the Table 3 and Figures 4 and 5 a comparison it shows between the means from area of ulcer, from area from ulcer in the eight week, the mean reduction, the area from reduced ulcer of, the percentage from closed ulcer, the closing velocity and percentage of patients with total closing at 9 weeks of treatment for each from two groups compared. Same in the above tables, a statistic comparison from mean values is included to each considered parameter. As shown in the P column from Table 3, statistically significant differences do not exist in any of measured parameters between the treatments of ulcers using the dermal equivalent generated using the supplement elaborated by the developed method and a conventional hydrocolloid dermal equivalent. Although statistically significant there is no decrease in the area from ulcers between both treatments, there is a significant reduction from approximately twice the size from wounds in the group of patients treated with the dermic equivalent. It is possible that can show significance to increase the number of patients in each group. ////

**Table 1. Comparison from general data of patients**

| | **Conventional Hydrocolloid Equivalent** | **Dermal Equivalent** | **Value from Statistic Test** | **P** |
|---|---|---|---|---|
| **Gender** | | | | |
| Females | 8 | 11 | 1.003 | 0.317 |
| Males | 10 | 7 | | |
| Total | 18 | 18 | | |
| **Age** | 58.61 +/- | 63.28 +/- | 1.13 | 0.264 |
| | 13.057 | 11.55 | | |
| **Evolution (months)** | | | | |
| **Mean** | 29.56 | 48.39 | 160 | 0.949 |
| **Median** | 8 | 9.5 | | |
| **Range** | 1-100 | 1-360 | | |
| **Consumption of NSAIDs** | 7 | 5 | 0.5 | 0.45 |
| **Comorbidities** | 1 | 5 | 1.8 | 0.177 |
| **Size of wound (cm2)** | | | | |
| **Mean** | 9.99 | 10.29 | 160.5 | 0.962 |
| **Median** | 5.67 | 6.24 | | |
| **Range** | 1 to 33 | 1 to 35 | | |

**Table 2. Comparison of general characteristics from ulcers.**

| | **Conventional Hydrocolloid Equivalent** | **Dermal Equivalent** | **Value from Statistic Test** | **P** |
|---|---|---|---|---|
| **Islands of epithelium (%)** | 1 | 3 | - | 0.603 |
| **Granulation (%)** | | | 157 | 0.873 |
| Mean | 73.33 | 72.22 | | |
| Median | 80 | 80 | | |
| Range | 20 to 100 | 0 to 100 | | |
| **Fibrin (%)** | | | 161 | 0.974 |
| Mean | 25 | 25 | | |
| Median | 20 | 15 | | |
| Range | 0 to 80 | 0 to 100 | | |
| **Exudate** | 15 | 16 | - | 1 |
| **Pain** | | | 159.5 | 0.937 |
| Mean | 5 | 5 | | |
| Median | 5.5 | 5.5 | | |
| Range | 0-10 | 0-10 | | |

**Table 3. Comparison from area of ulcer.**

| | **Conventional Hydrocolloid Equivalent** | **Dermal Equivalent** | **P** |
|---|---|---|---|
| **Basal (cm²)** | 9.99 | 10.29 | 0.96 |
| **Week 8 (cm²)** | 6.21 | 2.98 | 0.831 |
| **Media of reduction (cm²)** | 3.78 | 7.31 | 0.384 |
| **Area from reduced ulcer (cm²)** | 3.77 | 7.31 | 0.381 |
| **Percentage from closed ulcer (%)** | 66.3 | 75 | 0.235 |
| **Closing velocity (cm²/week)** | 0.47 | 0.91 | 0.613 |
| **% patients with total closing in 9 weeks** | 38.9 | 44.4 | 0.735 |

### EXAMPLE 7 - Comparison between the dermal equivalents from present invention and available commercially.

In the Table 4 the average weekly cost is presented from treatment using dermal equivalents available commercially and the cost of dermal equivalent elaborated using the supplement from present invention. The average cost from weekly treatment using the developed dermal equivalent is widely less to the average cost from conventional treatments; at the same time maintain the therapeutic activity. It is noteworthy that the treatment with Oasis is of lower price; however, does not have autologous cells such as the case from described in the developed dermal equivalent in accordance with the method described in the present invention. In the case of Dermalogen, is an acellular collagen matrix obtained from skin of cadaveric donators and is equal in costs to dermal equivalent described in accordance to the developed method in the present invention, On the other hand, its manufacture method is totally different to dermal equivalent described here.

**Table 4. Comparison of costs with other dermal equivalents**

| **COMPANY** | **TRADE NAME** | **AVERAGE COST OF WEEKLY TREATMENT BY CM2** |
|---|---|---|
| Integra Lifesciences Corp | Integra | $39 USD dls |
| Organogenesis | Apligraf | $31 USD dls |
| Organogenesis Inc | Dermagraft | $32 USD dls |
| BioHorizons | Alloderm | $33 USD dls |
| Collagen Matrix Technologies | Dermalogen | $18 USD dls |
| Smith & Nephew Inc. | Oasis wound matrix | $10 USD dls |
| - | Dermal equivalent elaborated in accordance with the developed method | $18 USD dls |

## Claims

1. A method for preparing a supplement to favor the *in vitro* cellular proliferation of cells of the cutaneous system, comprising the steps of:
a) cultivating mesenchymal cells of Wharton's jelly in a suitable culture medium with development factors to reach confluence, wherein said development factors are epidermal growth factor (EGF) and basic fibroblast growth factor (bFGF);
b) harvesting the cultivated cells of step a) and further cultivating said cells in DMEM 50% - F12 50% with bovine fetal serum with antibiotics, without development factors, to obtain a conditioned medium;
c) collecting the conditioned medium of step b);
d) repeating steps a) and b) at least once to obtain an enriched conditioned medium; and
e) concentrating the enriched conditioned medium of step d) and sterilizing by filtration to obtain the supplement.

2. A method to favor the development and activation *in vitro* of cells of the cutaneous system, comprising cultivating said cells in a culture medium containing the supplement prepared in accordance with claim 1.

3. The method in accordance with claim 2, **characterized in that** the cells of the cutaneous system comprise fibroblasts, keratinocytes, dendritic cells, monocytes or combinations of the same.

4. A method to manufacture a dermal equivalent, comprising the steps of:
- cultivating autologous or allogenic fibroblasts in a culture medium containing the supplement prepared in accordance with claim 1 to obtain a suitable confluence;
- harvesting by enzymatic treatment and washing the fibroblasts obtained in the previous step;
- reseeding the fibroblasts obtained in the previous step in a culture medium containing the supplement prepared in accordance with claim 1 at a supplement concentration of 5 - 50% with respect to the total volume of culture medium, and grow until a suitable confluence; and
- producing the dermal equivalent.

5. A method to manufacture a cutaneous equivalent, comprising the steps:
a) obtaining a dermal equivalent by the method of claim 4;
b) obtaining keratinocytes from an autologous or allogenic system;
c) cultivating the keratinocytes of step b) in a culture medium containing the supplement prepared in accordance with claim 1 to obtain a suitable confluence;
d) harvesting by enzymatic treatment and washing the keratinocytes obtained in step c);
e) reseeding the keratinocytes obtained in step d) in a culture medium containing the supplement prepared in accordance with claim 1 at a supplement concentration of 5 - 50% with respect to the total volume of culture medium, and grow until a suitable confluence; and
f) culturing the keratinocytes obtained in step 3) over the dermal equivalent of step a) to produce the cutaneous equivalent.

6. The method in accordance to claim 4, **characterized in that** the fibroblasts are from an autologous or allogenic system of human or animal origin.

7. The method in accordance to claim 5, **characterized in that** the keratinocytes are from an autologous or allogenic system from human or animal origin.

8. A method to manufacture a cellular suspension of fibroblasts, comprising the steps of:
a) culturing fibroblasts in a culture medium comprising the supplement prepared in accordance with claim 1 to reach a suitable cellular confluence;
b) separating by an enzymatic treatment and washing the fibroblasts obtained in step a) from the culture medium;
c) reseeding the fibroblasts obtained in step b) in a culture medium containing the supplement prepared in accordance with claim 1 at a supplement concentration of 5 - 50% with respect to the total volume of culture medium, and grow until a suitable confluence; and
d) incorporating the fibroblasts obtained in step c) in a solution of autologous blood serum.

## Patentansprüche

1. Verfahren zur Herstellung eines Supplements zur Begünstigung der Invitro-Zellproliferation von Zellen des kutanen Systems, umfassend die folgenden Schritte:
a) Kultivieren von mesenchymalen Zellen von Wharton-Sulze in einem geeigneten Kulturmedium mit Entwicklungsfaktoren, um Konfluenz zu erreichen, wobei die Entwicklungsfaktoren epidermaler Wachstumsfaktor (EGF) und basischer Fibroblasten-Wachstumsfaktor (bFGF) sind;
b) Gewinnen der kultivierten Zellen aus Schritt a) und weiteres Kultivieren der Zellen in DMEM 50 % - F12 50 % mit fötalem Rinderserum mit Antibiotika, ohne Entwicklungsfaktoren, um ein konditioniertes Medium zu erhalten;
c) Sammeln des konditionierten Mediums aus Schritt b);
d) Wiederholung der Schritte a) und b) mindestens einmal, um ein angereichertes konditioniertes Medium zu erhalten; und
e) Konzentrieren des angereicherten konditionierten Mediums aus Schritt d) und Sterilisieren durch Filtration, um das Supplement zu erhalten.

2. Verfahren zur Begünstigung der Entwicklung und Aktivierung von Zellen des kutanen Systems in vitro, umfassend das Kultivieren der Zellen in einem Kulturmedium, das das nach Anspruch 1 hergestellte Supplement enthält.

3. Verfahren nach Anspruch 2, **dadurch gekennzeichnet, dass** die Zellen des kutanen Systems Fibroblasten, Keratinozyten, dendritische Zellen, Monozyten oder Kombinationen davon umfassen.

4. Verfahren zur Herstellung eines dermalen Äquivalents, umfassend die Schritte:
- Kultivieren autologer oder allogener Fibroblasten in einem Kulturmedium, das den nach Anspruch 1 hergestellten Zusatz enthält, um eine geeignete Konfluenz zu erhalten;
- Gewinnen durch enzymatische Behandlung und Waschen der im vorhergehenden Schritt erhaltenen Fibroblasten;
- Wiedereinsäen der im vorhergehenden Schritt erhaltenen Fibroblasten in ein Kulturmedium, das das nach Anspruch 1 hergestellte Supplement in einer Supplementkonzentration von 5 - 50 % in Bezug auf das Gesamtvolumen des Kulturmediums enthält, und Wachsen bis zu einer geeigneten Konfluenz; und
- Erzeugen des dermalen Äquivalents.

5. Verfahren zur Herstellung eines kutanen Äquivalents, umfassend die Schritte:
a) Erhalten eines dermalen Äquivalents durch das Verfahren nach Anspruch 4;
b) Erhalten von Keratinozyten aus einem autologen oder allogenen System;
c) Kultivieren der Keratinozyten aus Schritt b) in einem Kulturmedium, das den nach Anspruch 1 hergestellten Zusatz enthält, um eine geeignete Konfluenz zu erhalten;
d) Gewinnen durch enzymatische Behandlung und Waschen der in Schritt c) erhaltenen Keratinozyten;
e) Wiedereinsäen der in Schritt d) erhaltenen Keratinozyten in ein Kulturmedium, das das nach Anspruch 1 hergestellte Supplement in einer Supplementkonzentration von 5 - 50 % in Bezug auf das Gesamtvolumen des Kulturmediums enthält, und Wachsen bis zu einer geeigneten Konfluenz; und
f) Kultivieren der in Schritt 3) erhaltenen Keratinozyten über dem dermalen Äquivalent von Schritt a), um das kutane Äquivalent zu erzeugen.

6. Verfahren nach Anspruch 4, **dadurch gekennzeichnet, dass** die Fibroblasten aus einem autologen oder allogenen System menschlichen oder tierischen Ursprungs stammen.

7. Verfahren nach Anspruch 5, **dadurch gekennzeichnet, dass** die Keratinozyten aus einem autologen oder allogenen System menschlichen oder tierischen Ursprungs stammen.

8. Verfahren zur Herstellung einer Zellsuspension von Fibroblasten, umfassend die folgenden Schritte:
a) Kultivieren von Fibroblasten in einem Kulturmedium, das das nach Anspruch 1 hergestellte Supplement umfasst, um eine geeignete zelluläre Konfluenz zu erreichen;
b) Abtrennen durch eine enzymatische Behandlung und Waschen der in Schritt a) erhaltenen Fibroblasten vom Kulturmedium;
c) Wiedereinsäen der in Schritt b) erhaltenen Fibroblasten in ein Kulturmedium, das das nach Anspruch 1 hergestellte Supplement in einer Supplementkonzentration von 5 - 50 % in Bezug auf das Gesamtvolumen des Kulturmediums enthält, und Wachsen bis zu einer geeigneten Konfluenz; und
d) Einbringen der in Schritt c) erhaltenen Fibroblasten in eine Lösung aus autologem Blutserum.

## Revendications

1. Procédé pour préparer un supplément pour favoriser la prolifération cellulaire *in vitro* de cellules du système cutané, comprenant les étapes consistant à :
a) cultiver des cellules mésenchymateuses de gélatine de Wharton dans un milieu de culture approprié avec des facteurs de développement pour atteindre la confluence, dans lequel lesdits facteurs de développement sont le facteur de croissance épidermique (EGF) et le facteur de croissance de fibroblastes basique (bFGF) ;
b) récolter les cellules cultivées de l'étape a) et cultiver additionnellement lesdites cellules dans du DMEM 50 % - F12 50 % avec du sérum fœtal bovin avec des antibiotiques, sans facteurs de développement, pour obtenir un milieu conditionné ;
c) recueillir le milieu conditionné de l'étape b) ;
d) répéter les étapes a) et b) au moins une fois pour obtenir un milieu conditionné enrichi ; et
e) concentrer le milieu conditionné enrichi de l'étape d) et le stériliser par filtration pour obtenir le supplément.

2. Procédé pour favoriser le développement et l'activation *in vitro* de cellules du système cutané, comprenant la culture desdites cellules dans un milieu de culture contenant le supplément préparé selon la revendication 1.

3. Procédé selon la revendication 2, **caractérisé en ce que** les cellules du système cutané comprennent des fibroblastes, des kératinocytes, des cellules dendritiques, des monocytes ou des combinaisons de celles-ci.

4. Procédé pour fabriquer un équivalent dermique, comprenant les étapes consistant à :
- cultiver des fibroblastes autologues ou allogéniques dans un milieu de culture contenant le supplément préparé selon la revendication 1 pour obtenir une confluence appropriée ;
- récolter par traitement enzymatique et laver les fibroblastes obtenus dans l'étape précédente ;
- ensemencer à nouveau les fibroblastes obtenus dans l'étape précédente dans un milieu de culture contenant le supplément préparé selon la revendication 1 à une concentration de supplément de 5 - 50 % par rapport au volume total de milieu de culture, et cultiver jusqu'à une confluence appropriée ; et
- produire l'équivalent dermique.

5. Procédé pour fabriquer un équivalent cutané, comprenant les étapes consistant à :
a) obtenir un équivalent dermique par le biais du procédé selon la revendication 4 ;
b) obtenir des kératinocytes à partir d'un système autologue ou allogénique ;
c) cultiver les kératinocytes de l'étape b) dans un milieu de culture contenant le supplément préparé selon la revendication 1 pour obtenir une confluence appropriée ;
d) récolter par traitement enzymatique et laver les kératinocytes obtenus dans l'étape c) ;
e) ensemencer à nouveau les kératinocytes obtenus dans l'étape d) dans un milieu de culture contenant le supplément préparé selon la revendication 1 à une concentration de supplément de 5 - 50 % par rapport au volume total de milieu de culture, et cultiver jusqu'à une confluence appropriée ; et
f) cultiver les kératinocytes obtenus dans l'étape 3) sur l'équivalent dermique de l'étape a) pour produire l'équivalent cutané.

6. Procédé selon la revendication 4, **caractérisé en ce que** les fibroblastes sont d'un système autologue ou allogénique d'origine humaine ou animale.

7. Procédé selon la revendication 5, **caractérisé en ce que** les kératinocytes sont d'un système autologue ou allogénique d'origine humaine ou animale.

8. Procédé pour fabriquer une suspension cellulaire de fibroblastes, comprenant les étapes consistant à :
a) cultiver des fibroblastes dans un milieu de culture comprenant le supplément préparé selon la revendication 1 pour atteindre une confluence cellulaire approprié ;
b) séparer par un traitement enzymatique et laver les fibroblastes obtenus dans l'étape a) à partir du milieu de culture ;
c) ensemencer à nouveau les fibroblastes obtenus dans l'étape b) dans un milieu de culture contenant le supplément préparé selon la revendication 1 à une concentration de supplément de 5 - 50 % par rapport au volume total de milieu de culture, et cultiver jusqu'à une confluence appropriée ; et
d) incorporer les fibroblastes obtenus dans l'étape c) dans une solution de sérum sanguin autologue.
